# EUROPÄISCHE PATENTSCHRIFT

(11) **EP 3 522 822 B1**
(45) Veröffentlichungstag und Bekanntmachung des Hinweises auf die Patenterteilung: **03.01.2024**
(21) Anmeldenummer: 17780383.0
(22) Anmeldetag: 04.10.2017
(51) Int. Cl.: A61C 19/045, A61C 9/00, A61C 13/00

(54) **HALTEBOGEN ZUR VERANKERUNG VON BEWEGUNGSSENSOREN UND VERFAHREN ZU DEREN HERSTELLUNG**
RETAINING ARC FOR ANCHORING MOTION SENSORS AND METHOD FOR MANUFACTURING SAME
ARC DE RETENUE DESTINÉ À L'ANCRAGE DE CAPTEURS DE MOUVEMENTS ET PROCÉDÉ DE FABRICATION

(30) Priorität: 04.10.2016 DE 102016012087
(43) Veröffentlichungstag der Anmeldung: 14.08.2019
(73) Patentinhaber: Forstgarten International Holding GmbH, 9016 St. Gallen (CH)
(72) Erfinder: HORNUNG, Frank, 97779 Geroda (DE); KALTENBACH, Stefan, 9445 Rebstein (CH); WEIHE, Stephan, 45478 Mühlheim an der Ruhr (DE)
(74) Vertreter: Schmidt, Christian
(86) Internationale Anmeldenummer: PCT/EP2017/075252
(87) Internationale Veröffentlichungsnummer: WO 2018/065487

(56) Entgegenhaltungen:
- WO-A2-2014/044783
- DE-A1- 3 700 142
- DE-U1-202014 103 131
- DE-U1-202017 101 547
- KR-A- 20110 104 230

## Beschreibung

### Gebiet der Erfindung

Die vorliegende Erfindung betrifft eine Vorrichtung zur reversibel lösbaren Befestigung an Zähnen eines Oberkiefers oder eines Unterkiefers, wobei ermöglicht ist, dass der Patient eine ungehinderte Kaubewegung durchführt, welche z. B. durch weitere Elemente, die an der Vorrichtung angebracht sind, überwacht, aufgezeichnet und analysiert werden kann. Ferner ist ein entsprechendes Herstellungsverfahren bereitgestellt.

### Stand der Technik

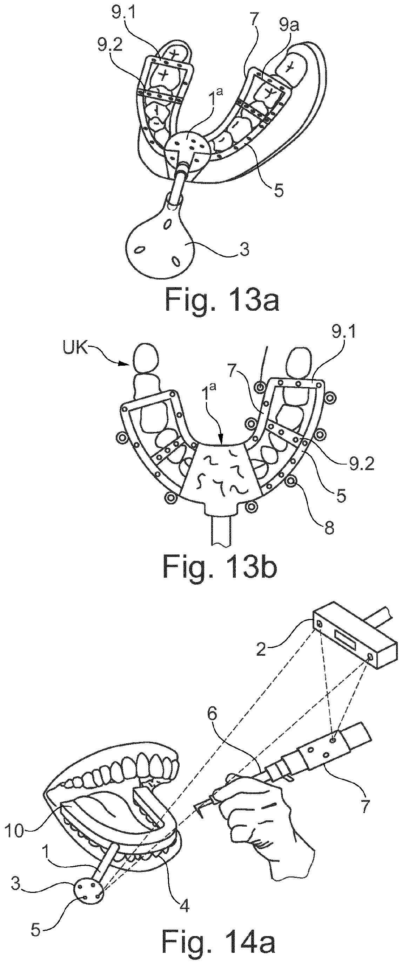

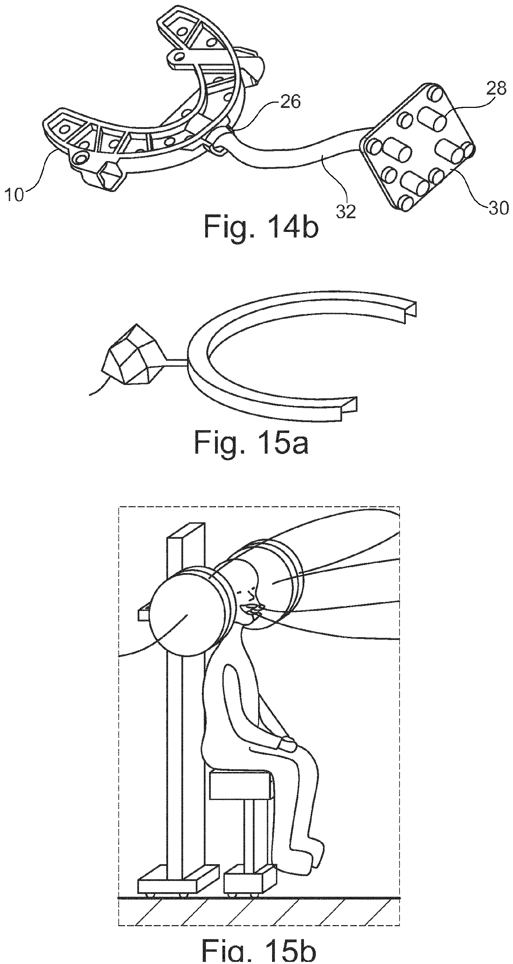

Vorrichtungen zum Verankern von Messvorrichtungen sind dem Fachmann bekannt:
DE 202014102968 U1 beschreibt einen Kopplungslöffel zur temporären Fixierung von Unterkiefer-Mess-Sensorik am Unterkiefer, UK, eines Wirbeltiers, ausgebildet als im Wesentlichen starres, kastenförmiges Formteil, insbesondere Kunststoff-Formteil, zum Einbringen von UK-Befestigungsmasse zur Befestigung an den Vorderzähnen des UK, welches auf seiner Oberseite eine zum leichten Gleitender Vorderzähne des Oberkiefers, OK, ausgebildete Gleitfläche aufweist, sowie mit Markierungen, welche für einen 3D-Oberflächenscanner mit einem gegenüber der Bügel-Längserstreckung kleineren Erfassungsbereich erfassbar und derart ausgebildet und angeordnet sind (siehe Fig. 14a,b). Bei diesen und anderen bekannten Technologien liegen Teile der Kopplungslöffel so, dass sie die Kauflächen teils oder auch ganz überdecken.

US 7457443 B2 beschreibt ein Verfahren und eine Vorrichtung zur komplexen Beseitigung von Erfassungsfehlern, um die Position der Kiefer noch genauer messen und verfolgen zu können. (Siehe Fig. 15a,b). Die Marker, die am Kiefer befestigt werden, sind besonders gestaltet, um besser erkannt zu werden. Doch auch hier überdeckt der Kopplungslöffel, das Attachment, die Okklusion.

DE 102009027356 A1 nennt ein bildgebendes System zur Erzeugung eines 3D-Datensatzes (siehe Fig. 16a,b) mindestens eines Teils eines Kiefers mittels Bilderzeugungseinheit zur dreidimensionalen Vermessung eines in einem Untersuchungsvolumen befindlichen Objekts, dadurch gekennzeichnet, dass ein Messapparat vorhanden ist, der während einer Aufnahme oder einer Aufnahmeserie der Bilderzeugungseinheit mindestens ein Signal (S) erzeugt, das der relativen Lage des Oberkiefers (37) und des Unterkiefers zueinander mindestens in einer Richtung der Beweglichkeit entspricht und dass eine Zuordnung von den mit Bilderzeugungseinheit (11) erzeugten Aufnahmen zu dem mindestens einen Signal (S) erfolgt. Dabei wird ein okklusaler Löffel gezeigt, mit dessen Hilfe die Befestigung unter Verwendung von Abformmasse erfolgt.

DE 37 00 142 A1 beschreibt eine an der unterkiefer-Zahnreihe eines Patienten befestigbare Halterung für ein zahnärztliches Gerät.

DE 20 2014 103131 U1 beschreibt einen paraokklusalen Bügel zur temporären Fixierung von Unterkiefer-Messsensorik am Unterkiefer eines Wirbeltiers.

WO 2014/044783 A2 beschreibt ein Verfahren zur Simulation der dynamischen Okklusion.

Insbesondere sind im Stand der Technik keine Haltevorrichtungen verwendet worden, welche es ermöglichen, eine natürliche Kaubewegung aufzuzeichnen, zu überwachen bzw. zu analysieren.

Das Problem ist dabei, dass durch die Verdeckung der Okklusion der biomechanisch extrem wichtige Schlussbiss gar nicht mehr hergestellt werden kann.

Sämtliche Vorrichtung nach dem Stand der Technik haben ein oder mehrere der folgenden Nachteile:
a) sie verdecken ganz oder teilweise die Okklusionsflächen und erlauben keinen direkten Schlussbiss,
b) sie benötigen entweder Klebstoff oder mechanische fixierte Verbindungen direkt an den Zähnen und sind nachher nicht leicht entfernbar,
c) sie erreichen nicht die geforderte Genauigkeit und Reproduzierbarkeit der Positions- und Bewegungsmessung wegen unerwünschter Relativbewegungen zwischen Zahnbogen und Haltevorrichtung.

Die Aufgabe der Erfindung ist es, eine stabile und leicht einsetzbare und leicht lösbare Befestigungsvorrichtung zu bieten, die einen ungehinderten Schlussbiss erlaubt. Oberstes Ziel ist dabei, die Okklusionsflächen am Zahnbogen frei lassen. Es handelt sich also um ein Non-Okklusales-Attachment, das am Zahnbogen und damit am Kiefer eines Probenanden oder Patienten befestigt werden kann.

Die weitere Aufgabe der Erfindung besteht darin, das Haltemoment gegen relative Verlagerung des Haltebogens gegenüber dem Zahnbogen deutlich höher auszubilden, als es mit elastischen Abformmassen bisher möglich war.

### Zusammenfassung der Erfindung

Die Aufgabe wird erfindungsgemäß gelöst durch eine Vorrichtung und ein Herstellungsverfahren gemäß den unabhängigen Ansprüchen. Bevorzugte Ausführungsbeispiele finden sich in den untergeordneten Ansprüchen.

Gemäß einer Ausführungsform der vorliegenden Erfindung ist bereitgestellt eine Vorrichtung zur reversibel lösbaren Befestigung an Zähnen eines Oberkiefers oder Unterkiefers, aufweisend: konkave Oberflächenbereiche, die jeweils zum Kontaktieren von einem lateralen, insbesondere bukkalen, Oberflächenbereich eines (modifizierten) Zahnes ausgebildet sind, um jeweils einen Formschluss und/oder einen Kraftschluss zu bilden, wobei Kauflächen sämtlicher Zähne frei bleiben, sodass eine Kaubewegung nicht behindert ist.

Die Vorrichtung kann wiederholt an den Zähnen befestigt werden, und von diesen wiederum gelöst werden. Um die Vorrichtung zu befestigen, ist kein Klebstoff oder Klebemittel erforderlich. Ein Lösen der Vorrichtung erfordert nicht ein Aufbrechen von Klebestellen. Nach dem Entfernen der vorgeschlagenen Vorrichtung von den Zähnen müssen die Zähne zweckmäßigerweise nicht nachbearbeitet werden, zum Beispiel durch Polieren. Wird eine Klebeverbindung zwischen der Vorrichtung und dem Zahn zur Befestigung genutzt, so müssen Klebstoff- oder Klebemittelreste meist nachträglich entfernt werden, was vorliegend nicht erforderlich ist. Im Wesentlichen (wenn auch nicht notwendigerweise ausschließlich, aber doch möglicherweise ausschließlich) erfolgt die Befestigung der Vorrichtung über einen Formschluss und/oder Kraftschluss der konkaven Oberflächenbereiche der Vorrichtung mit den lateralen (insbesondere konvexen) Oberflächenbereichen der (modifizierten) Zähne. Ein Stoffschluss, etwa durch einen Klebstoff vermittelt, ist mit Vorteil nicht an der Befestigung beteiligt. Bei den Zähnen kann es sich um natürliche oder künstliche Zähne handeln. Auf den Zähnen, insbesondere den lateralen Oberflächenbereichen der Zähne, können (müssen jedoch nicht) weitere Elemente aufgebracht sein, etwa durch Aufkleben von Höckern, um eine Konvexität der Oberflächenbereiche der Zähne zu erhöhen. Die konkaven Oberflächenbereiche der Vorrichtung können komplementär zu konvexen Oberflächenbereichen der Zähne ausgebildet sein. Dadurch kann ein effektiver Formschluss erreicht werden, insbesondere sofern eine ausreichende Normalkraft ausgeübt ist, welche auf die konkaven Oberflächenbereiche der Vorrichtung zu dem jeweiligen Oberflächenbereich des Zahnes hin wirkt. Wegen der freibleibenden Kauflächen wird der Schlussbiss oder die natürliche Okklusion nicht ver- oder behindert. Dies hat erhebliche biomechanische Vorteile.

Die konkaven Oberflächenbereiche können jeweils beispielsweise eine Flächenausdehnung zwischen 0,5 mm² und 10 mm² haben. Andere Werte sind möglich. Gemäß einer Ausführungsform der vorliegenden Erfindung beträgt eine Ausdehnung des (oder eines von mehreren) konkaven Oberflächenbereichs der Vorrichtung zwischen 30 % und 80 % einer Flächenausdehnung der gesamten lateralen Fläche des Zahnes, welchen der betreffende konkave Oberflächenbereich im befestigten Zustand der Vorrichtung kontaktiert. Je größer die Flächenausdehnung des konkaven Oberflächenbereichs ist, der den jeweiligen Oberflächenbereich des Zahnes kontaktiert, umso höher kann die Befestigungskraft sein, mit der die Vorrichtung an den Zähnen befestigt ist.

Damit kann die Vorrichtung beispielsweise als Hilfs- bzw. Haltevorrichtung für Komponenten eines Systems zum Aufzeichnen und Analysieren einer Kaubewegung des Patienten verwendet werden.

Die konkaven Oberflächenbereiche können ausschließlich, gemäß einer Ausführungsform, die bukkalen (d. h. wangenseitigen) Oberflächenbereiche der Zähne kontaktieren. In anderen Ausführungsformen kann beispielsweise ein Zahnstumpf umgriffen werden, sodass sowohl bukkale als auch linguale (d. h. zungenseitige) Bereiche des Stumpfes gewisse Bereiche der Vorrichtung kontaktieren.

Die Kauflächen der Zähne sind diejenigen Oberflächenteile der Zähne, welche während eines Kauvorgangs mit (gegenüberliegenden) Zahnoberflächen in Kontakt kommen. Die Kauflächen werden innerhalb dieser Anmeldung mitunter auch als Okklusionsflächen bezeichnet, da diese Flächen die Kontaktbereiche während eines Verschlusszustandes des Gebisses, d. h. eines Beißzustandes, darstellen. Die Vorrichtung kann ferner ausgebildet sein, auch einen sich horizontal (bukkal und/oder lingual) an die Kauflächen anschließenden Bereich freizulassen, welcher Bereich nämlich während einer natürlichen Kaubewegung von gegenüberliegenden Zähnen zeitweise eingenommen werden kann.

Gemäß einer Ausführungsform der vorliegenden Erfindung kann die am Oberkiefer befestigbare Vorrichtung, im am Oberkiefer befestigten Zustand, vollständig oberhalb der Beißebene liegen. Gemäß einer Ausführungsform der vorliegenden Erfindung kann die am Unterkiefer befestigbare Vorrichtung, im am Unterkiefer befestigten Zustand, vollständig unterhalb der Beißebene liegen. Somit kann eine ungehinderte Kaubewegung gewährleistet werden.

Die Vorrichtung kann als ein Bogen ausgebildet sein und weist insbesondere im Wesentlichen eine U-Form, z. B. eine Hufeisenform, auf. Die Vorrichtung kann insbesondere außen umlaufend um die Zähne ausgebildet sein. Die Oberflächenbereiche der Vorrichtung und die Oberflächenbereiche der Zähne können jeweils durch eine elastomechanische Kraft in Kontakt gebracht werden und mittels der elastomechanischen Kraft gehalten werden. Zum Anbringen an die Zähne kann die Vorrichtung an den Enden der "U"s leicht aufgebogen werden bzw. gespreizt werden, im gespreizten Zustand im Gebiss positioniert werden und dann kann die Spreizkraft nachgelassen werden, sodass die Enden der Vorrichtung bzw. die beiden sich daran anschließenden Arme der Vorrichtung aufgrund der elastomechanischen Kraft in Kontakt mit den lateralen Oberflächenbereichen der Zähne gebracht werden und einen Formschluss bilden.

Die Oberflächenbereiche der Vorrichtung und die Oberflächenbereiche der Zähne können zumindest abschnittsweise komplementäre Oberflächenformen aufweisen. Dadurch kann ein effektiver Formschluss gebildet werden.

Gemäß einer Ausführungsform der vorliegenden Erfindung weisen die Oberflächenbereiche der Vorrichtung zumindest drei, insbesondere vier, konkave Oberflächenbereiche (insbesondere jeweils für einen Zahn oder Stumpf) der Vorrichtung auf, insbesondere einen linken vorderen, einen linken hinteren, einen rechten vorderen und einen rechten hinteren Oberflächenbereich. Dabei bezieht sich links, rechts bzw. vorne, hinten auf Bereiche des Unterkiefers bzw. des Oberkiefers, welche somit auch entsprechende Bereiche der Vorrichtung im befestigten Zustand definieren. Wenn zumindest drei konkave Oberflächenbereiche der Vorrichtung vorgesehen sind, welche mit entsprechenden drei lateralen Oberflächenbereiche von drei Zähnen in Kontakt gebracht werden und einen Formschluss bilden, kann eine stabile Befestigung der Vorrichtung erreicht werden. Die Vorrichtung kann mehr als vier konkave Oberflächenbereiche aufweisen, welche mit jeweiligen lateralen Oberflächenbereichen der Zähne in Kontakt kommen und jeweils einen Formschluss bilden. Damit kann die Festigkeit der Befestigung weiter erhöht werden. Zwischen den vorderen (z. B. den linken vorderen oder den rechten vorderen) und den hinteren (z. B. den linken hinteren oder den rechten hinteren) Oberflächenbereichen kann jeweils ein Zwischenabschnitt der Vorrichtung vorgesehen sein, in dem keine Kontaktbereiche zu Zähnen vorhanden sind oder nur form- und/oder kraftschlussfreie Kontaktbereiche. Damit kann erreicht werden, dass die Vorrichtung im Formschluss an definierten Positionen verankert ist, welche einen gewissen (Mindest-)Abstand voneinander aufweisen, sodass eine stabile Befestigung erreicht werden kann. Andernfalls könnte aufgrund von Ungenauigkeiten einer Fertigung der Vorrichtung, ein Kontaktbereich nahe bei einem weiteren Kontaktbereich liegen, wobei jedoch ein vorgesehener weiter entfernt liegender Kontaktbereich aufgrund einer ungenauen Fertigung nicht tatsächlich im Kontakt mit den lateralen Oberflächenbereichen der Zähne steht. In diesem Fall wäre keine stabile Befestigung erreicht.

Ein Abstand zwischen den vorderen und hinteren Oberflächenbereichen kann jeweils 0,1 bis 0,9, insbesondere 0,2 bis 0,6, mal eine Gebissausdehnung von vorne nach hinten betragen. Je größer der Abstand ist, umso effektiver und stabiler kann die Befestigung erfolgen. Die Oberflächenbereiche der Vorrichtung können z. B. derart ausgebildet und angeordnet sein, dass sie z. B. mit einem Eckzahn und dem letzten oder vorletzten Backenzahn in Kontakt kommen. Je nach Grad einer Konvexität der Zähne und den Abständen zwischen den Zähnen können geeignete laterale Oberflächenbereiche der Zähne ausgewählt werden, welche wiederum die komplementär dazu geformten konkaven Oberflächenbereiche der Vorrichtung definieren können.

Einer der Oberflächenbereiche, insbesondere alle der Oberflächenbereiche der Vorrichtung, können konkav in zwei quer, insbesondere senkrecht, zu einander verlaufenden Richtungen sein. Wenn die Oberflächenbereiche bzw. der zumindest eine Oberflächenbereich konkav in zwei nicht parallelen Richtungen ist, so kann der Formschluss eine Haltekraft in zwei quer zueinander laufenden, insbesondere senkrecht zueinander laufenden Richtungen bewirken. Damit kann eine stabile Befestigung erreicht werden. Je nach Anatomie und Form der Zähne können entsprechend geformte Kontaktbereiche an den Zähnen ermittelt und ausgewählt werden, wodurch auch die komplementären Oberflächenbereiche der Vorrichtung in Lage und Form definiert sein können.

Die Vorrichtung kann ferner eine Befestigungsmöglichkeit für ein Element, insbesondere eine Markierung und/oder einen Signalgeber und/oder einen Sensor und/oder eine Lichtquelle und/oder einen Bügel bereitstellen. Damit kann die Vorrichtung z. B. zur Analyse einer Kaubewegung verwendet werden, wobei sichergestellt ist, dass eine natürliche Kaubewegung ungehindert durchgeführt werden kann. Bei der Markierung kann es sich um eine optische Markierung handeln, bei dem Sensor kann es sich z. B. um einen Gyrosensor handeln und an dem Bügel können wiederum Markierungen bzw. Marker befestigt werden, welche zur Analyse der Geometrie und/oder der Bewegung des Gebisses verwendet werden können. Ferner kann an der Vorrichtung ein Signalgeber oder -empfänger, z. B. ein Radiosender und -empfänger, angebracht sein, welcher z. B. die von dem Sensor ermittelten Daten an eine andere Systemkomponente übermitteln kann.

Die Befestigungsmöglichkeit kann als ein im Bereich der Schneidezähne angeordneter nach vorne hervorstehender Arm ausgebildet sein. Damit kann der Arm, ohne eine Kaubewegung zu behindern, durch die Lippen nach außen geführt werden, um ungehindert andere Elemente an der Vorrichtung anbringen zu können.

Die Vorrichtung kann mehrteilig, insbesondere zweiteilig, ausgebildet sein und ferner mindestens ein Kopplungselement aufweisen, mit dessen Hilfe die mehreren Teile der Vorrichtung gekoppelt werden können, während die Vorrichtung an den Zähnen befestigt ist. Dabei kann durch Koppelung der mehreren Teile der Vorrichtung eine Anpresskraft auf die Oberflächenbereiche der Vorrichtung ausgeübt werden, die diese an die lateralen Oberflächenbereiche der Zähne presst, sodass der Formschluss erfolgt. Für diese Ausführungsform kann die Vorrichtung aus einem rigiden, im Wesentlichen nicht elastischen Material, gebildet sein. Ferner kann ein Anbringen der Vorrichtung bei einer Mehrteiligkeit vereinfacht sein. Ferner kann eine Anpresskraft zwischen den konkaven Oberflächenbereichen der Vorrichtung und den lateralen Oberflächenbereichen der Zähne durch ein geeignetes Kopplungselement, beispielsweise eine Schraube, eingestellt werden, sodass eine Befestigungskraft gemäß einem Zielwert eingestellt werden kann.

Die Vorrichtung kann einteilig ausgeführt sein.

Die Vorrichtung kann, bereichsweise oder insgesamt, elastisch verformbar sein. Mit Vorzug ist die Vorrichtung in einem Bereich elastisch verformbar, der den Formschluss oder Kraftschluss zu einem Zahn herstellen soll. Die Befestigung der Vorrichtung an dem jeweiligen Zahn oder den jeweiligen Zähnen kann durch eine elastische Rückstellkraft gestärkt werden.

Die Vorrichtung kann aus einem elastischen Material, insbesondere Kunststoff, gefertigt sein. Andere Materialien sind möglich. Die Vorrichtung muss nicht notwendigerweise aus einem elastischen Material gebildet sein. Das elastische Material kann eine elastische Verformbarkeit der Vorrichtung fördern oder verursachen. Die elastischen Rückstellkräfte, die die Vorrichtung nach Verformung wieder in die Ursprungsform zurückversetzen wollen, können die Befestigung der Vorrichtung an dem jeweiligen Zahn stärken. Der Zahn ist dann mit Vorzug das Element, das verhindert, dass ein elastisch verformter Bereich der Vorrichtung wieder die ursprünglich, nicht-deformierte Form annimmt. Die Vorrichtung ist zweckmäßigerweise dazu vorgesehen im elastischen Verformungsbereich des elastischen Materials eingesetzt zu werden und mit Vorzug nicht im Bereich der plastischen Verformung, der eine permanente Verformung bedingen würde, die keine elastischen Rückstellkräfte mehr nach sich ziehen würde. Das elastische Material kann ein Thermoplast sein.

Vorrichtungen aus elastischem Material können die einteilige Ausführung der Vorrichtung begünstigen.

Gemäß einer Ausführungsform der vorliegenden Erfindung ist ferner bereitgestellt ein Verfahren zur Herstellung einer Vorrichtung zur reversibel lösbaren Befestigung an Zähnen eines Oberkiefers oder Unterkiefers, wobei das Verfahren aufweist: Bilden von konkaven Oberflächenbereichen, die jeweils zum Kontaktieren von einem lateralen, insbesondere bukkalen, Oberflächenbereich eines (möglicherweise modifizierten) Zahnes ausgebildet sind, um jeweils einen Formschluss und/oder Kraftschluss zu bilden, wobei Kauflächen sämtlicher Zähne frei bleiben, sodass eine Kaubewegung nicht behindert ist.

Im Zusammenhang mit der Vorrichtung genannte Merkmale können ebenso für das Verfahren verwendet werden und umgekehrt.

Das Verfahren kann teilweise in Software und teilweise in Hardware implementiert werden. Insbesondere kann eine Visualisierungs- oder CAD-Software verwendet werden, mithilfe derer eine Form der Vorrichtung definiert werden kann. Das Verfahren kann ferner ein Bereitstellen eines 3D-Modells der Zähne aufweisen, das die lateralen zur Kontaktierung mit der Vorrichtung vorgesehenen Oberflächenbereiche der Zähne des Oberkiefers oder des Unterkiefers umfasst. Das 3D-Modell kann insbesondere alle Zähne des Oberkiefers bzw. des Unterkiefers umfassen, sowie auch Bereiche des Zahnfleischs. Das Verfahren kann ferner ein Modifizieren des 3D-Modell durch Vergrößern in Bereichen außerhalb der zur Kontaktierung vorgesehenen lateralen Oberflächenbereiche umfassen. Diese nicht zur Kontaktierung vorgesehenen Oberflächenbereiche können somit durch Aufbringen (virtuell oder real) von einer gewissen Schichtdicke definiert werden. Die Form der Vorrichtung kann durch Komplementbildung des modifizierten Modells definiert werden. Die Komplementbildung kann real oder virtuell erfolgen.

Das Definieren der Form der Vorrichtung kann ferner ausgehend von einem (z.B. U-förmigen) Rohling erfolgen. Der Rohling kann im Wesentlichen eine U-Form, z.B. eine Hufeisenform, aufweisen. Während der Herstellung können aus dem Rohling (real oder virtuell) Raumbereiche ausgeschnitten werden, welche dem modifizierten 3D-Modell der Zähne entsprechen.

Das Modifizieren des 3D-Modells kann ein Definieren eines Abstands von nicht zur Kontaktierung vorgesehenen Oberflächenbereichen der Zähne zu nicht zur Kontaktierung vorgesehenen Oberflächenbereichen der Vorrichtung zwischen 0,1 mm und 2 mm umfassen. Dadurch können gezielt in einem Zwischenbereich zwischen den Kontaktbereichen Kontaktierungen zwischen Bereichen der Vorrichtung und lateralen Oberflächenbereichen der Zähne vermieden werden, um eine Verankerung durch Formschluss an definierten Kontaktbereichen zu bewirken, welche einen gewissen Abstand voneinander haben.

Das 3D-Modell kann als reales körperliches Objekt oder als elektronischer Datensatz bereitgestellt sein. Das Modifizieren kann am realen körperlichen Objekt oder unter Zuhilfenahme einer den elektronischen Datensatz darstellenden Visualisierungssoftware erfolgen. Das Definieren der Form der Vorrichtung kann durch physikalischen Abdruck des modifizierten Modells oder Komplementbildung bzw. Differenzbildung zwischen dem Rohling und dem modifizierten Modell in einer elektronischen Datenverarbeitungsanlage erfolgen.

Somit kann das Verfahren teilweise virtuell und teilweise in der physikalischen Realität durchgeführt werden. Das Verfahren kann ferner ein Ausgeben eines die Form der Vorrichtung definierenden Datensatzes an einen 3D-Drucker aufweisen, sowie Herstellen der Vorrichtung mittels des 3D-Druckers. Auch andere Systeme zur additiven Fertigung können zur Herstellung der Vorrichtung eingesetzt werden.

### Kurze Figurenbeschreibung

Die Figuren zeigen Ausführungsformen der vorliegenden Erfindung. Die Erfindung ist nicht auf die illustrierten oder beschriebenen Ausführungsformen beschränkt.
Figur 1 a,b zeigen eine schematische Darstellung von Zahnbogen (1) und Haltebogen (4) mit Schnittstelle (5) sowie mit den Klemm-Kontaktstellen (41.1 ... 41.4). Rechts die non-okklusale Anordnung mit oben frei bleibender Okklusionsfläche (12);
Figur 2 a,b zeigt Links: Zahnbogen (1) und Kiefer (2) als virtuelles 3D Modell (3) mit virtuellen Okklusionsflächen 32 und bukkalen Kontaktflächen 31 (31.1. bis 31.4); Rechts: Zahnoberfläche im 3D Modell mit Kontaktfläche (31.1) im hinteren Kontaktbereich (44). Oben die Okklusionsflächen (1.6) und Nebenzahn (1.5). Das Kreuz markiert den Kraftschwerpunkt des Anpressdruckes, den der Haltebogen (4) mit Kontaktfläche (41.1) am Zahnbogen ausüben wird;
Figur 3 zeigt einen Zahnbogen (1) als Ausschnitt mit Markierungen für Kontaktelemente (41.1) und (41.2) sowie aufgebrachter Abstandsschicht (46.1). Durch die Abstandssschicht (46) liegt der Haltebogen (4) nur in den Bereichen (43) und (44) am Zahnbogen auf. Dadurch entsteht eine 4-Punkt Befestigung. Der Abstand der Auflagebereiche vorne und hinten ist der Hebelarm HA (45) gegen vertikale Auslenkungen bei Belastung des Halteelements;
Figur 4 zeigt einen Haltebogen (4) aus gedrucktem Kunststoff mit hoher elastischer Festigkeit und geringer plastischer Verformbarkeit. Die Kontaktflächen 41.1 bis 41.4. liegen im hinteren Molarenbereich und im Eckzahnbereich. Dadurch wird die Haltevorrichtung (4) optimal gegenüber des Zahnbogens (1) stabilisiert. An der mechanischen Schnittstelle (5) wird das Instrumentarium befestigt;
Figur 5 zeigt einen Eckzahn mit Haltebogen in direktem Kontakt. Die Okklusion 12 ist oben unbehindert. Der Presskontakt der Kontaktfläche 41.1 wurde erreicht, indem die 3D Form am Kontakt direkt ohne Veränderung aus dem 3D Model 31.1. genommen wurde. Zahn 1.6, Wurzel 1.61 und Zahnfleisch 1.62.
Figur 6 zeigt einen Vorderen Molar mit Haltebogen mit Spalt ohne Kontakt, Die Okklusion 12 ist oben auch hier unbehindert. Der Spalt der Kontaktfläche 41.7 wurde erreicht, indem die 3D Form am Kontakt erst durch eine Veränderung 3D Model 31.7. umgearbeitet wurde. Dann erst wurde das umgearbeitete 3D Modell zur Herstellung des 3D Modells 40 des Haltebogens verwendet. Der Haltebogen hat am gezeigten Zahn den Abstand (41.7-31.7)

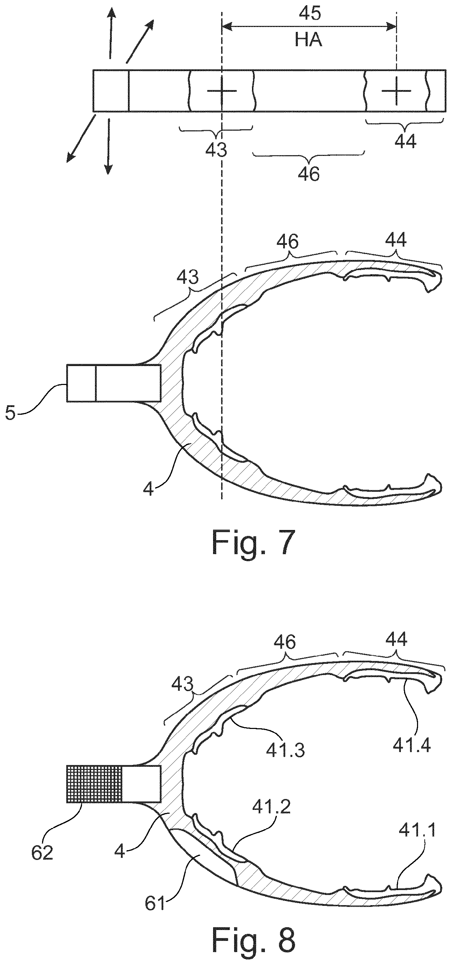

Figur 7 zeigt einen Haltebogen (4) in Seitenansicht und Aufsicht. Mit den vorderen Kontaktbereichen (43) und den hinteren Kontaktbereichen (44) und dazwischen liegend den Abstandsbereichen (46) mit Abstand HA (45). Die eingeleitete Last an der mechanischen Schnittstelle (5) wird als Kraft auf die Kontraktbereiche (43) und (44) übertragen;
Figur 8 zeigt einen Haltebogen (4) zusätzlich mit Aufnahmevorrichtungen für Sensorelemente (61) oder Marker (62);
Figur 9 zeigt ein 3D Modell (3) des Zahnbogens, mit Kennzeichnung der geplanten Kontaktbereiche (31.1 ... 31.4), an denen der Haltebogen mit Kontaktflächen (41.1.... 41.4) anliegen wird. In den dazwischen liegenden Abstandsbereichen wird ein abstand (33.1 und 33.2) gezielt aufgebracht und anschließend mit dem 3D Modell zum veränderten 3D Modell (30) umgearbeitet. Das veränderte 3D Modell (30) steuert anschließend die Berechnung des virtuellen Haltebogens (40) und die Produktion des realen Haltebogens (4);
Figur 10 zeigt ein virtuelles 3D-Modell des Haltebogens (40) als Steuerdatensatz für die Produktion des realen Haltebogens (4) hergestellt aus Überlagerung des veränderten virtuellen Zahnbogens (30) mit einem virtuellen Haltebogen Preform (70);

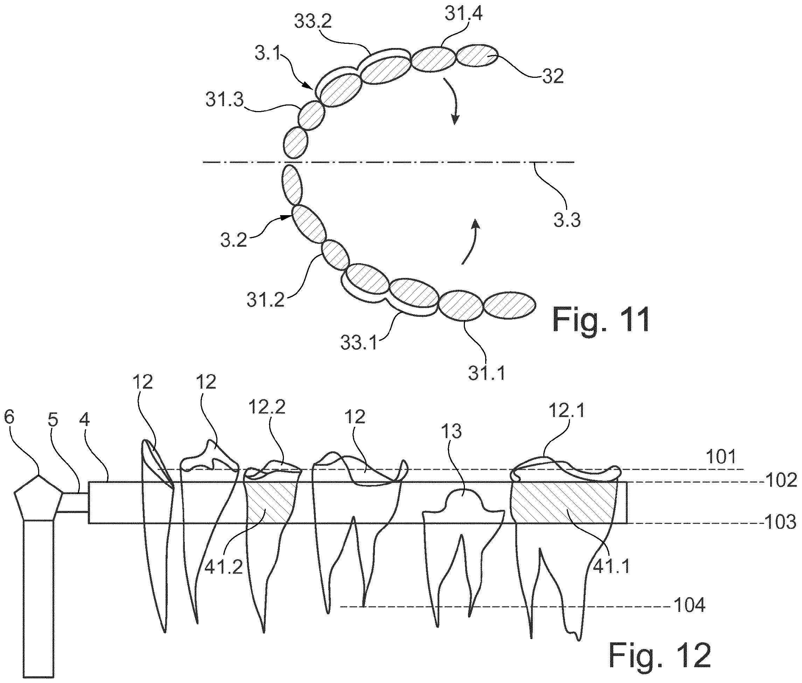

Figur 11 zeigt ein zweigeteiltes virtuelles 3D Modell des Zahnbogens (3). Die eine Hälfte 3.1 und die zweite Hälfte (3.2.) werden gezielt gegeneinander verlagert, so dass sich ein reduzierter Abstand zwischen 31.1. und 31.4 ergibt. Dies erzeugt eine verstärkte elastische Deformation des Haltebogens (4), sobald er auf den Zahnbigen (1) aufgesteckt wird;
Figur 12 zeigt eine Darstellung der Non-Okklusalen Ausführung des Haltebogens (40). Der Haltebogen (4) klemmt im Mund am Zahnbogen (1) des Unterkiefers (2) und trägt an der mechanischen Schnittstelle (5) den Sensorapparat (6). Gemessen wird mit optischen Methoden die Relativbewegung des Unterkiefers zum Oberkiefer, also die Bewegung im Kiefergelenk;
Figur 13 zeigt den Stand der Technik mit einem Löffel, der die Okklusionsflächen verdeckt und den Schlussbiss verhindert
Figur 14 zeigt den Stand der Technik mit einem Marker, der die Positionsbestimmung des Unterkiefers erlaubt, jedoch den biomechanisch wichtigen Schlussbiss behindert;
Figur 15 zeigt den Stand der Technik mit einem angedockten Marker zur Messung mit einem Abformlöffel, der den Kontakt des Unterkiefers mit dem Oberkiefer verhindert;
Figur 16 a bis f zeigen eine erfindungsgemäße Herstellung des virtuellen 3D Haltebogens (40) anhand von 3D Daten (3) von Zahnbogen (1) und Gebiss (2) sowie Abstandsmittel im Bereich (46) zwischen den Klemm-Kontaktflächen (43) und (44);
Figur 17 a,b zeigt einen realen Haltebogen (4) aus Kunststoff, hergestellt in einem 3D Drucker, mit Retentionsflächen (41) und mechanischer Schnittstelle (5);

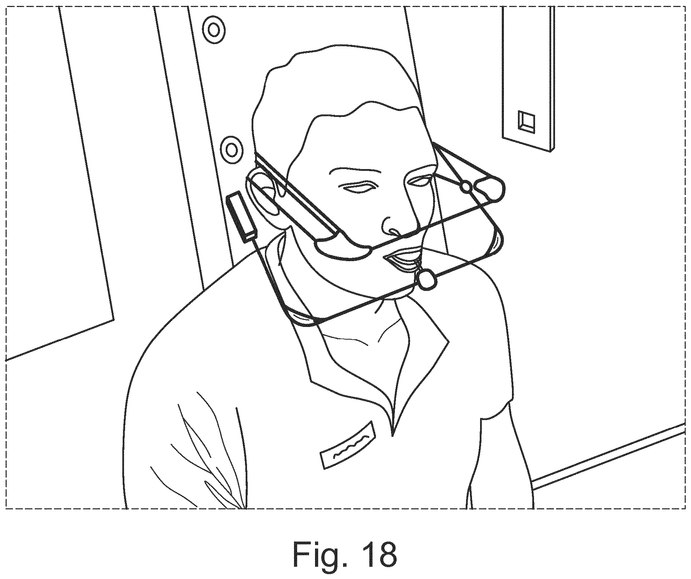

Figur 18 zeigt einen Probanden mit eingesetztem Haltebogen (4) und an der Schnittstelle (5) angesetzter Messvorrichtung mit Markern (6).

### Detaillierte Beschreibung

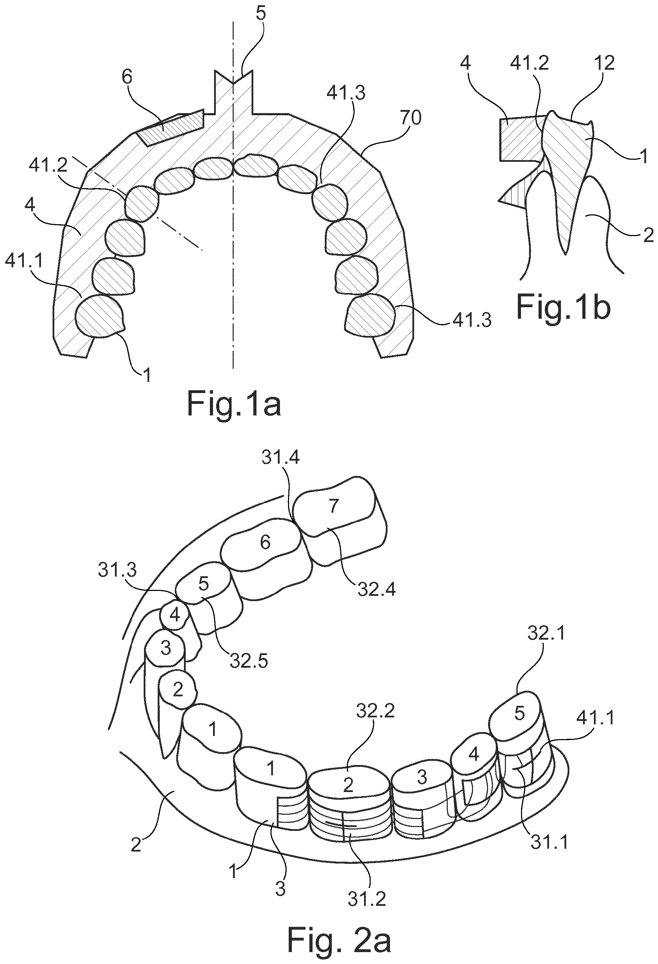

Figuren 1a, 1b illustrieren z. B. in einer Draufsicht eine Vorrichtung 70, 4 zur reversibel lösbaren Befestigung an Zähnen 1 eines Oberkiefers oder eines Unterkiefers. Die Vorrichtung 70, welche auch als Haltebogen bezeichnet wird, umfasst dabei konkave Oberflächenbereiche 41.1, 41.2, 41.3, 41.4, die mit lateralen bukkalen Oberflächenbereichen gewisser Zähne 1 im Kontakt stehen und einen Formschluss bilden. Wie in der Figur 1b in einer Seitenansicht schematisch illustriert ist, sind Kauflächen 12 (auch als Okklusionsfläche bezeichnet) durch die lateral außen angebrachte Vorrichtung 4 freigelassen, sodass eine Kaubewegung nicht behindert ist.

Wie insbesondere aus Figur 1b ersichtlich ist, liegt, für einen Unterkiefer, die Vorrichtung 4 vollständig unterhalb einer Kauebene, sodass auch ein seitliches Ausweichen von in Figur 1b nicht illustrierten, gegenüberliegenden Zähnen während einer Kaubewegung durch die angebrachte Vorrichtung 4 nicht behindert ist.

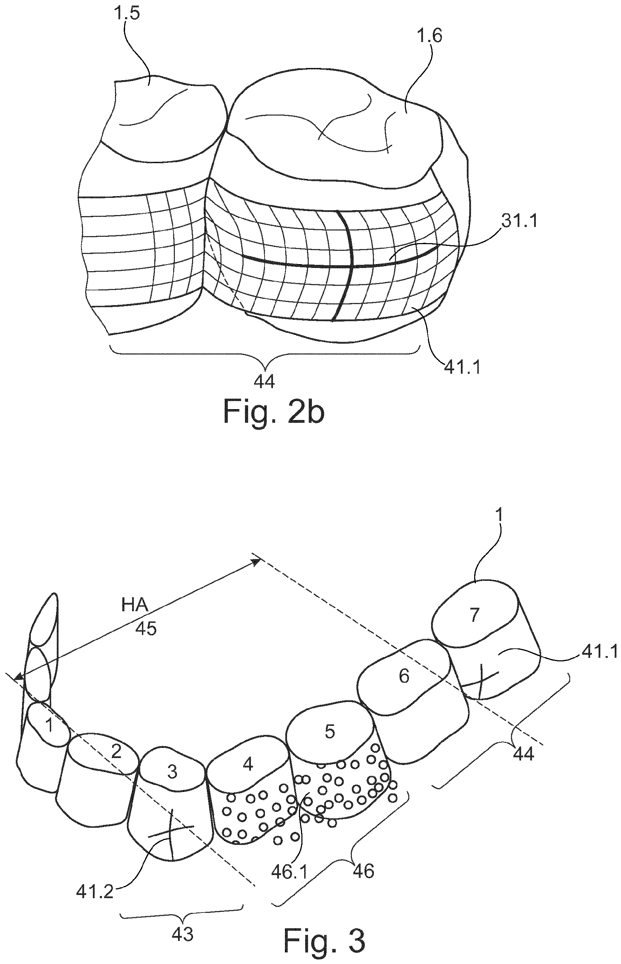

Wie in Figur 2b illustriert ist, umfasst ein Zahn in dem Bereich 44 einen Oberflächenbereich 41.1, welcher in zwei nahezu senkrecht aufeinander stehende Richtungen (illustriert durch fette Linien) eine konvexe Form aufweist. Die Vorrichtung 70 bzw. 4 weist in diesem Bereich eine komplementäre konkave Form auf, sodass die Vorrichtung in diesem Oberflächenbereich konkav in zwei nahezu senkrecht aufeinander stehenden Richtungen gebildet ist. Dadurch kann ein Formschluss verbessert werden.

Wie ferner in Figur 3 in einer schematischen perspektivischen Darstellung illustriert ist, befinden sich in Bereichen 43, 44 konkave Oberflächenbereiche der Vorrichtung, welche mit konvexen lateralen bukkalen Oberflächenbereichen 41.1, 41.2 der Zähne in Kontakt stehen. In dem Zwischenbereich 46 steht die Vorrichtung nicht in Kontakt mit den lateralen Außenoberflächen der Zähne 4 und 5.

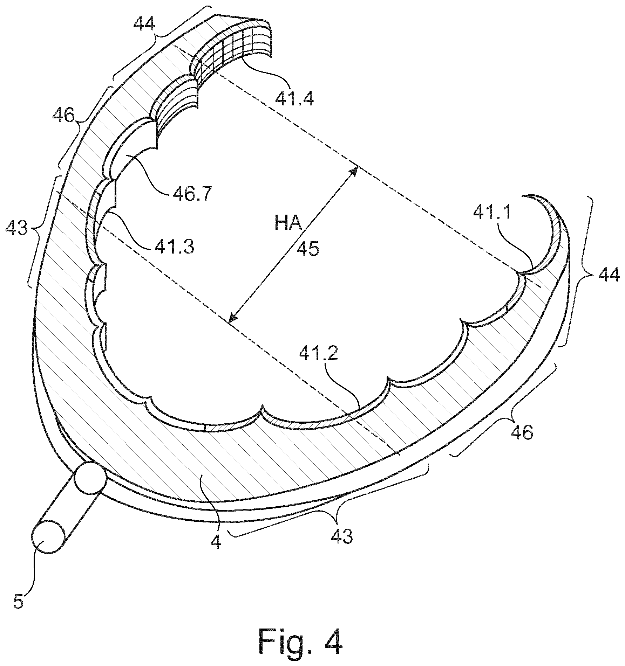

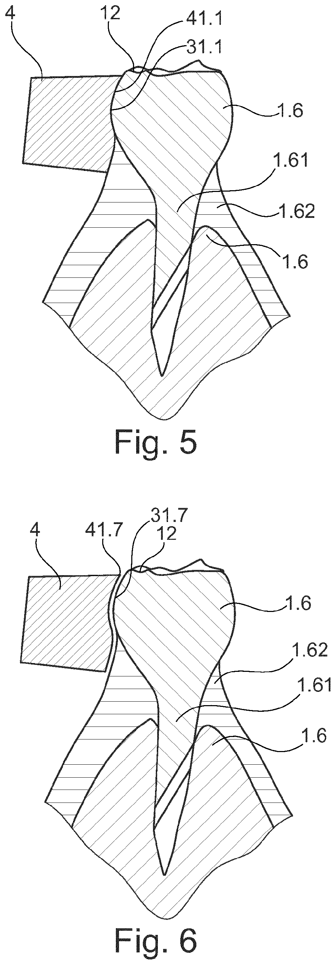

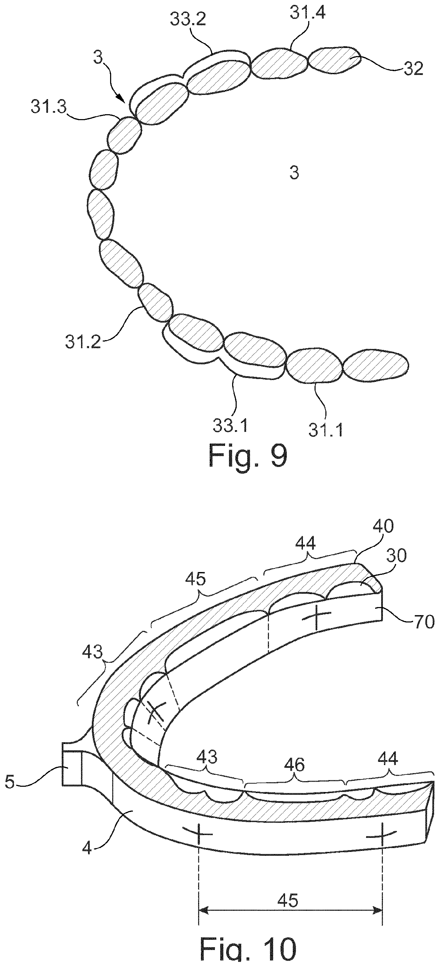

Der Abstand zwischen vorderen und hinteren Oberflächenbereichen der Vorrichtung, die mit lateralen Oberflächenbereichen der Zähne im Kontakt stehen, kann HA betragen, wie beispielsweise in Figur 4 illustriert ist. Während in den konkaven Oberflächenbereichen 41.1, 41.2, 41.3, 41.4 die Vorrichtung 4 in Kontakt mit der konvexen lateralen Außenoberfläche 31.1 des Zahnes steht (siehe Figur 5), ist in dem Zwischenbereich 46 ein Spalt zwischen der lateralen Außenoberfläche 31.7 des Zahnes und der Oberfläche 41.7 der Vorrichtung 4 gebildet (siehe Fig. 6). Um diesen Spalt in einer gefertigten Vorrichtung 4 zu erhalten, kann z. B. (virtuell oder in Realität) eine gewisse Schicht 33.1, 33.2 auf diejenigen Zähne eines 3D-Modells aufgebracht werden, deren Außenoberflächen zur Befestigung der Vorrichtung nicht kontaktiert werden sollen, wie beispielsweise in Figur 9 illustriert ist.

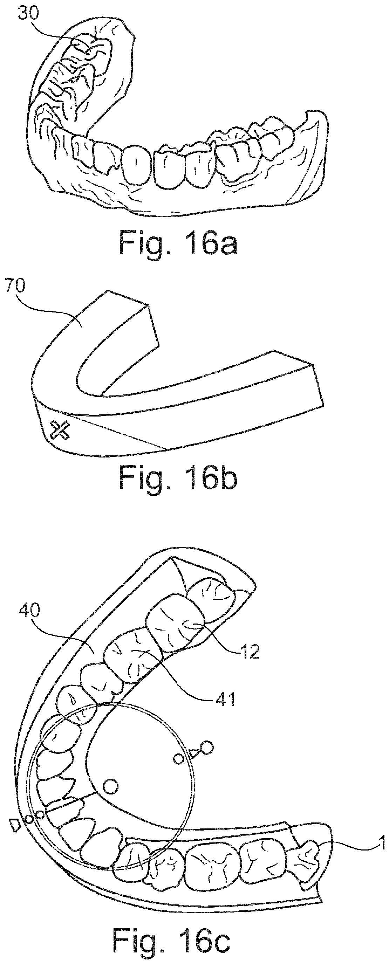

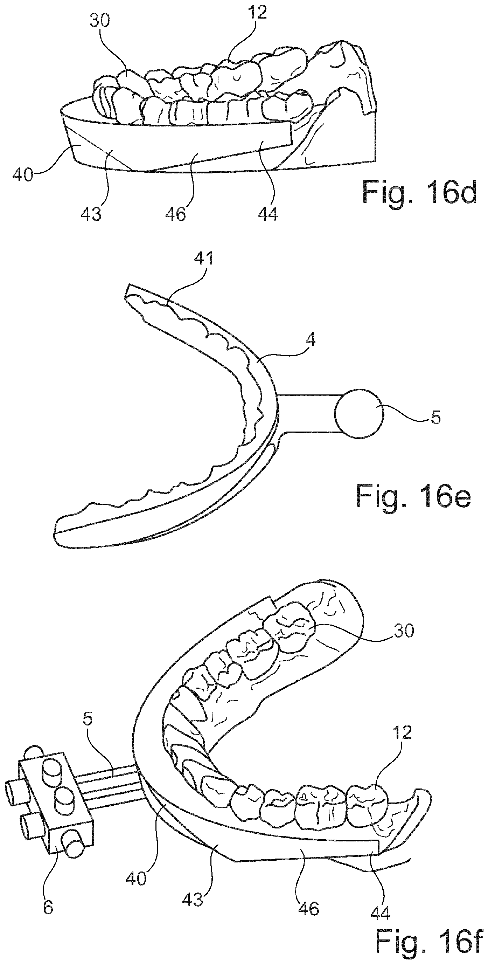

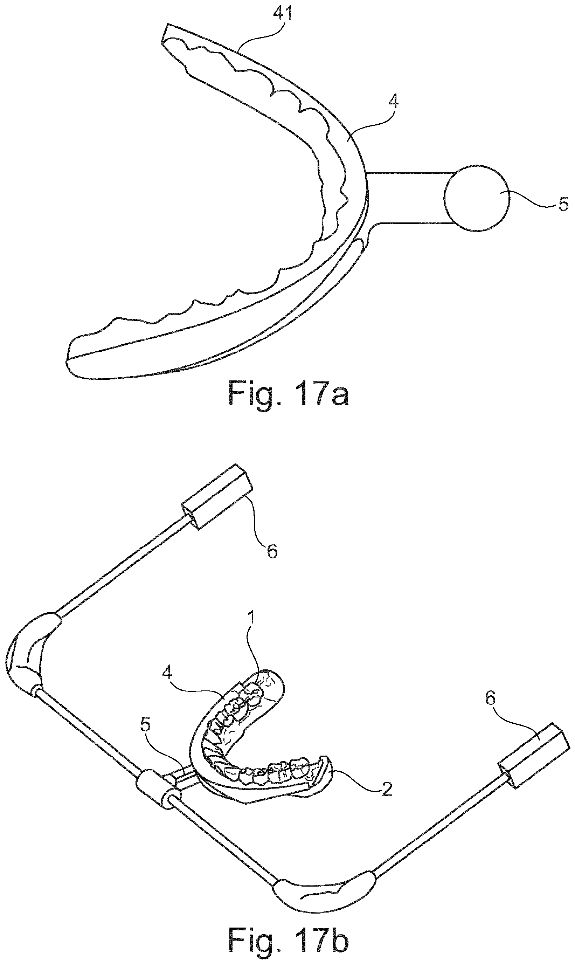

An die Vorrichtung können verschiedene Elemente angebracht werden, wie in Figuren 13a, 13b, 14a, 14b, 15a schematisch illustriert ist. Damit können Untersuchungen am Gebiss, insbesondere Untersuchungen zu einer Kaubewegung, durchgeführt werden. Wie z. B. in Figur 16f illustriert ist, kann an die Vorrichtung eine Lichtquelle 6 angebracht werden oder etwa ein Bügel mit weiteren Elementen, wie in Figur 17b illustriert ist.

Die Erfindung betrifft ferner die reversible und schonende Befestigung einer Haltevorrichtung (4) an einem Zahnbogen (1), mit Hilfe von non-okklusalen lateralen Kontaktflächen (41.1 ... 41.4) in einem Kiefer (2), wobei alle Okklusionsflächen (12) der Zähne frei und ungehindert verbleiben.

Die Erfindung umfasst des Weiteren einem anhand dieser realen Vorlage (1) hergestelltem 3D Modell (3) des Zahnbogens mit den darin enthaltenen Objekten wie Zähnen und Implantaten sowie dessen virtuelle Veränderung, um einen besseren Klemmsitz für den Haltebogen (4) zu erreichen.

Die besondere Schwierigkeit der Aufgabe liegt darin, dass - wenn man die Okklusionsflächen (12) frei lässt - nur noch die lateralen Oberflächenbereiche der Zähne (31) bzw. in Realität (11) und insbesondere der Eckzähne und Molaren für eine Befestigung des Haltebogens (4) zur Verfügung stehen.

Mit Hilfe eines Bildgebungsverfahrens wird ein 3D Modell des Zahnbogens einschließlich Zahnfleisch erstellt. Dies erfolgt entweder durch direkte bildgebende Verfahren oder durch Abbildung eines Abgusses oder einer Abformung, insbesondere durch Röntgenstrahlen.

Der virtuelle 3D Zahnbogen (3) hat eine Reihe von Okklusionsflächen (32) die vollständig frei und unbehindert bleiben müssen, damit die sinnvollen Messungen, die Okklusion erfordern, überhaupt durchgeführt werden können. Mit den bisher verfügbaren Befestigungsvorrichtungen und Löffel-Konstruktionen war dies nicht der Fall.

Die erfindungsgemäße Vorrichtung kann aus einem elastomechanisch belastbarem Haltebogen (4) aus biegefestem Material mit einer mechanisch Klemmkraft ausübenden Kontaktflächenanordnung (41) bestehen, die auf mindestens 3, bevorzugt 4 konkave Kontaktflächenbereiche (41.1 bis 41.n), verteilt ist.

Der Haltebogen klemmt am Zahnbogen (1) fest und positionsgenau mit transversaler Klemmkraft zwischen den beiden hinteren Haltebereichen (44), gebildet aus (41.1 und 41.4) und zwischen den beiden vorderen Haltebereichen (43), gebildet aus (41.2 und 42.3). Der Abstand der Kraftzentren des hinteren (44) und vorderen (43) Haltebereichs bildet den Hebelarm HA (45).

Die Positionsgenauigkeit ergibt sich aus lokal an den Kontaktbereichen 41 konkav geformten Oberflächenelementen 41 (41.1 ... 41.n), die zu den konvex geformten bukkalen bzw. auch frontalen Zahnoberflächen 31 (31.1 ... 3 1.n) kongruent sind.

Die Okklusionsflächen (1.5. und 1.6) des Zahnbogens (1) bleiben gezielt frei und ermöglichen den Schlussbiss auch bei eingesetztem Haltebogen (4).

Die Kontaktflächen (41) können durch Einsetzen des Haltebogens (4) an das korrespondierende Gebiss (1) positionsgenau in Klemmkontakt mit einem Zahnbogen (1) gebracht werden. Am Haltebogen (4) befindet sich eine mechanische Schnittstelle (5) zur Anbringung von Instrumenten oder Sensoren.

Dabei sind zwischen den zum Zahnbogen formkongruenten Kontaktflächenbereichen (41) definierte Abstandsflächenbereiche (46, z.B. 46.7) vorhanden, die gezielt nicht in Kontakt sondern in leichtem Abstand mit dem Zahnbogen (1) stehen.

Zwischen dem hinteren (44) und dem vorderen Auflagebereich (43) befindet sich beiderseits ein Abstandsbereich (46), indem der Haltebogen (4) nicht oder nur mit wenig Kontaktkraft auf dem Zahnbogen (3) anliegt. Die Schwerpunkte der Kontaktflächenanordnung (41) bilden dabei eine vordere Kontaktgruppe (43) im Eckzahnbereich und eine hintere Kontaktgruppe (44) im Molarenbereich.

Der erfindungsgemäße Haltebogen (4) lässt die Okklusionsflächen (12) des Zahnbogens (1) komplett frei und beschränkt den Kontaktbereich auf die non-okklusalen Oberflächen (11) des Zahnbogens (1) und des Kieferbogens (2). Der Haltebogen erstreckt sich in okklusaler Richtung bis knapp unter die Okklusionsebene, in Richtung Zahnwurzel bis fast an das Zahnfleisch oder erreicht auch das Zahnfleisch in einigen Bereichen.

Im Bereich der Kontaktflächen (41.1) liegen diese direkt am Zahn (1.1) an, gemäß 3D Formfläche (31.1) Im Bereich der nicht krafttragenden Abstandsfläche (41.7) jedoch besteht ein Spalt zwischen der Fläche (41.7) im Haltebogen und dem Zahnbogen mit Fläche (31.7).

Der Haltebogen (4) wird über den kraftschlüssigen und formschlüssigen Presskontakt an den Kontaktflächen (41) mittels elastomechanischer Klemmkraft am Zahnbogen (1) befestigt.

Die Klemmung zwischen dem vorderen Bereich (43) und dem hinteren Bereich (44) erzeugt über den Abstand (45) als langem Hebelarm ein besonders hohes Verankerungsmoment mit hoher Belastbarkeit und sehr guter Positionsstabilität insbesondere gegen transversale und vertikale Belastungen über die mechanische Schnittstelle (5), an der z.B. externe Marker angebracht sind.

Wenn wie gemäß Stand der Technik der Klemmbogen ohne die Abstandsbereiche (46) aufgebracht würde, würde sich die transversale Klemmung nicht über einen so großen Hebelarm HA (45) zwischen den Auflagerbereiche (43) und (44) erstrecken und das Haltemoment wäre deutlich geringer.

In einer besonderen Ausführung beinhaltet der Haltebogen eine Vorrichtung für das direkte Anbringen von Sensoren (61) oder Sendern (62) zum Zweck der Positions- und Bewegungserfassung, insbesondere des Unterkiefers relativ zum Oberkiefer.

Zwischen vorderer (43) und hinterer Kontaktgruppe (44) besteht ein als Hebelarm (45) wirkender Abstand HA von bevorzugt mehr als 25mm. Im Gegensatz dazu lag bei bisherigen Abformungen von zahnbögen genau im Bereich 46 die maximale Auflagekraft. Haltebögen aus Abformmaterial schwingen daher um die Achse zwischen den Bereichen (46) und sind mechanisch kippstabil.

Zur Herstellung des anschließend zu fertigenden Haltebogens (4) verwendet die erfindungsgemäße Technologie nicht wie bisher mit einer möglichst genauen Abformung und Nachbildung der bukkalen 3D Flächen (11.1 .... 11.n) des Zahnbogens (1) sondern mit einer ganz gezielten Abweichung (33) in den Abstandsbereichen (46) zwischen den Kontaktbereichen (43) und (44).

Zur Herstellung des Haltebogens (4) wird eine virtuelle 3D Zahnbogenmodell (3) des Zahnbogens (1) als Ausgangsbasis verwendet. Der reale Zahnbogen (1) umfasst die okklusalen Kauflächen (12), die mittels der virtuellen Okklusionsflächen (32) abgebildet in 3D digital sind.

In den Kontaktflächenbereichen (31.1 .. 31.4) liegt das 3D Modell möglichst genau am 3D Datenmodell des Zahnbogens (1). Mit verfügbarer Technik können Genauigkeiten zwischen 10 und 100 Mikron erreicht werden. Dazwischen in den Kontaktbereichen (31) befinden sich die Abstandsbereiche (33.1 und 33.2).

An diesen Abstandsbereichen befindet sich das virtuelle 3D für den Haltebogen weiter bukkal, erzeugt z.B. durch eine Auflageschicht (33.1 und 33.2), die virtuell auf diese dort liegenden Zahnbereiche aufgetragen wird. Der Abstand der Formfläche 33.2 von der unveränderten virtuellen 3D Zahnbogenfläche 3 beträgt ca. 0,1 bis 1 mm.

Beim Einsetzen des Haltebogens (4) auf den Zahnbogen (1) wird der Haltebogen im hinteren Bereich (44) stärker elastisch aufgeweitet als im vorderen Haltebereich (43). Dadurch wird hinten die erforderliche Spannkraft erreicht und der Haltebogen transversal in der Ebene des Zahnbogens verriegelt. Die konkave Form der Kontaktflächen in den Haltebereichen (43 und 44) erzeugt über die lokalen Retentionen die Verriegelung der Haltebogens in den beiden dazu senkrechten Raumrichtungen. Durch die Kombination mehrerer Lagerpunkte (41.1 ... 41.4) wird die Verriegelung des Haltebogens relativ zum zahnbogen in allen 6 Raumdimensionen erreicht (3 Freiheitsgrade Rotation und 3 Freiheitsgrade Translation).

Die non-okklusale Ausführung des Haltebogens (4) beinhaltet die Anordnung des Bogens entlang einer transversalen Ebene durch die Klemm-Kontaktflächen 41.1 und 41.2. Die Okklusionsflächen 11 liegen oberhalb des Haltebogens (4).

Gegenüber dem Stand der Technik lässt sich dank der erfindungsgemäßen Vorrichtung die Lokalisierung bzw. Bewegungsverfolgung des Unterkiefers im Vergleich zum Oberkiefer erheblich genauer und besser reproduzierbar erfassen und verfolgen. Dies gilt auch dann, wenn die Vorrichtung zwischendurch entnommen und dann wieder eingesetzt wird.

Die Erfindung wird unabhängig davon verwirklicht, wie die Anordnung mit vorderen Auflagebereichen (43) und hinteren Auflagebereichen (44) hergestellt wird. Wesentliches Merkmal ist die 6D Verschlüsselung durch die angepressten konkaven Kontaktflächen (41) des Haltebogens (4). Dies garantiert die exakte und dauerhaft stabile Befestigung von Instrumenten, Sendern, Markern oder Bewegungssensoren etc.

### Ausführungsbeispiel: Erzeugung des virtuellen 3D Haltebogens

### INPUT X

□ Oraler SCAN des Gebisses mit ORAL SCANER (3)
   Geräte: 3 Shape, Sirona, EMS, ...
   Format STL
□ Desktop SCAN des Modells
   Geräte: 3 Shape, Sirona, CadStar
   Format STL
□ 3D Aufnahme eines Volumentomografen (Dicom Format) des Modells oder des Abformlöffels umgewandelt in STL Format
   Geräte: CT oder DVT
   Format: STL

### INPUT Y

Vorgeformter virtueller 3D Halbling (70) für den Haltebogen
□ Rohling des Non Oklussalen Löffels, ggf. schon dimiensioniert auf Patienten Anatomie, Unterkieferzahnbogen Form.
   Format: STL

### ZWISCHENERGEBNIS

□ Erweiterung des Zahnbogens (3) durch die Abstandsbereiche (46) mit Abstandsmitteln
□ Verschmelzung zum angepassten Zahnbogen-Modell (30)

### OUTPUT

□ Boolsche Subtraktionsmenge als Kontur des Haltebogens (40)
□ zahnseitig mit den Flächen des zur optimalen Klemmung angepassten virtuellen Zahnbogens (30) und
□ oben, unten und außen mit den Flächen des Haltebogen-Halblings (70)

### Fig. 16 a, b, c

links: angepasster 3D Zahnbogen (30) und Mitte: virtueller 3D Bogen-Halbling (70) passend zur Kiefergeometrie (2)
Rechts: Überlagerung von Zahnbogen (30) und Bogen-Halbling (70)

### Fig. 16 d,e,f

links: virtueller Haltebogen (40) mit Kontaktbereichen (43) und (44) angepasster 3D Zahnbogen (30) wobei die okklusalen Flächen des Zahnbogens völlig frei bleiben
Mitte: virtueller 3D Haltebogen (40) als Datensatz zur Herstellung des realen Haltebogens mit den zahnseitigen Klemm-Kontaktflächen (41.1 ... 41.4)
rechts: Virtuelle Darstellung von Zahnbogen (30) und Haltebogen (40) mit mechanischer Schnittstelle (5) und Marker (6)

### Fig. 17 a,b

Der Haltebogen (4) klemmt fest sitzend am Zahnbogen (1) und stützt sich auf dem Kiefer (2) mit Zahnfleisch ab. An der Schnittstelle (5) ist eine Messapparatur (6) befestigt. Im Bild eine groß bauende sehr präzise Ausführung zur optischen Positionsbestimmung mit Hilfe von passiven Markern.

### Fig. 18

Der Haltebogen (4) klemmt im Mund am Zahnbogen (1) des Unterkiefers (2) und trägt an der mechanischen Schnittstelle (5) den Sensorapparat (6). Gemessen wird mit optischen Methoden die Relativbewegung des Unterkiefers zum Oberkiefer, also die Bewegung im Kiefergelenk.

### Weitere Ausführungsbeispiele:

Ausgehend von einem 3D Datenmodell (3) von Zahnbogen (1) und Unterkiefer (2) wird zunächst virtuelle bukkal eine Schicht mit Dicke 0,2 bis 0,5mm im Bereich der vorderen Molaren aufgetragen. Dann wird diese Schicht im 3D Modell mit dem Zahnbogen verschmolzen und es entsteht das gezielt veränderte 3D Modell des Zahnbogens (30). Dann wird der veränderte virtuelle 3D Zahnbogen (30) mit dem Preform (70) als Bogenelement zum Schnitt gebracht. Dadurch entsteht das virtuelle 3D Modell (40) des veränderten Haltebogens. Mittels 3D Drucker wird das virtuelle 3D Model (40) ausgedruckt. Die Kanten und spitzen werden digital 3D oder am 3D Ausdruck entgratet. Danach erfolgt eine Sterilisation. Danach kann der Haltebogen für Bewegungsmessungen am Patienten oder bei Probanden mehrfach eingesetzt werden. Bei Bedarf kann ein neuer identischer Haltebogen gedruckt werden.

In einem alternativen Ausführungsbeispiel wird im 3D Datenmodell des Zahnbogens (3) das Volumen der insbesondere im Abstandsbereich (46) liegenden Zähne virtuell 3D aufgeblasen, so dass das angepasste 3D Datenmodell des Zahnbogens (30) dort einen Abstandsspalt (33) zum unveränderten 3D Zahnbogen (3) aufweist. Der Abstand des veränderten 3D Modells (30) beträgt 0,2 bis 0,5mm im Bereich der vorderen Molaren (46). Nun wird der so veränderte virtuelle 3D Zahnbogen (30) mit dem Preform (70) als Bogenelement zum Schnitt gebracht. Dadurch entsteht das virtuelle 3D Modell (40) des veränderten Haltebogens.

In einem alternativen Ausführungsbeispiel wird das 3D Modell (3) des Zahnbogens geteilt und eine Hälfte (3.1) wird relativ zur anderen Hälfte (3.2) um einen kleinen Winkel verkippt oder um eine kleine Strecke verschoben, so dass im hinteren Kontaktbereich (44) der Abstand zwischen den Kontaktflächen (31) bzw. (41) geringer wird. Dann werden die beiden Hälften (3.1) und (3.2) zu einem angepassten 3D Modell (30) des Zahnbogens verschmolzen. Der Abstand des veränderten 3D Modells (30) beträgt nach elastischer Deformation 0,0 bis 0,5mm insbesondere im Bereich der vorderen Molaren (46). Nun wird der so veränderte virtuelle 3D Zahnbogen (30) mit dem Preform (70) als Bogenelement zum Schnitt gebracht. Dadurch entsteht das virtuelle 3D Modell (40) des veränderten Haltebogens.

In einer besonders bevorzugten Ausführung wird der virtuelle 3D Zahnbogen (3) in weiten Bereichen durch Aufbringen virtueller Deckschichten oder durch aufblasen der 3D Volumina der Zähne erweitert und nachträglich werden in den Haltebogen (4) an den Bereichen (43) und (44) gegenüber der Kontaktflächen (31) maßgenaue Kontaktflächenelemente (48.1 ... 48.4) eingebracht, die den formschlüssigen und kraftübertragenden Klemmkontakt herstellen.

Das digitale 3D Modell (40) des Haltebogens wird mit Hilfe von direkter oder indirekter 3D Formgebung maßgenau in den realen Haltebogen (4) umgesetzt. Technisch verfügbar sind z.B. 3D Druck, Laserpolymerisation, 3D Fräsen etc. Die Erfindung erstreckt sich auch auf andere Formgebungsverfahren.

Ferner werden die nachfolgend genannten Ausführungsformen bereitgestellt, wobei der Schutzbereich durch den Inhalt der Ansprüche bestimmt ist. Sofern die nachfolgend genannten Ausführungsformen so interpretiert werden sollten, dass sie Gegenstände betreffen, die nicht von den anhängenden unabhängigen Ansprüchen geschützt werden, so sind die Ausführungsformen so auszulegen, dass sie alle weiteren, in den anhängenden unabhängigen Ansprüchen definierten Merkmale aufweisen, die über die Merkmale der Ausführungsformen hinausgehen. Daher sind die nachfolgend genannten Ausführungsformen von den anhängenden unabhängigen Ansprüchen abgedeckt.

Eine erste Ausführungsform einer Vorrichtung zur lagegenauen Verankerung von Objekten an einem Zahnbogen (1) eines Probanden zur Erfassung von Position und oder Bewegung des Zahnbogens (1) bzw. des Kiefers (2), insbesondere des Unterkiefers relativ zum Oberkiefer, umfasst folgende Merkmale: eine non-okklusale Ausführung als lateral umlaufender Haltebogen (4) mit freien Okklusionsflächen (12) des Zahnbogens (1) und Kontaktflächen (41) an den lateralen bukkalen Zahnaußenflächen; eine Fixierung des Haltebogens (4) relativ zu Zahnbogen (1) und Kiefer mit Zahnfleisch (2) durch elastomechanische Kräfte, die die Kontaktflächen des Haltebogens (41) gegen die Kontaktflächen des Zahnbogens (11) drücken; und eine Verschlüsselung der Position gegen Rotation und Translation durch die an den konkaven Kontaktflächen und Retentionen (41) der Haltebogens (4) wirkenden Presskräfte.

Eine zweite Ausführungsform der Vorrichtung, umfasst die Merkmale der ersten Ausführungsform zur präzisen räumlichen Verankerung am Zahnbogen, sowie folgende weitere Merkmale: mindestens 3 Lagerpunkte an Kontaktflächen (41.1 ... 41.3), die eine Ebene aufspannen; und konkave Kontaktflächen am Zahnbogen (11) und am Haltebogen (41) in zwei Dimensionen mit Retentionen sowohl in der Schnittlinie mit der aufgespannten Ebene als auch senkrecht zur Schnittlinie und zur aufgespannten Ebene.

Eine dritte Ausführungsform der Vorrichtung umfasst die Merkmale der ersten oder der zweiten Ausführungsform . Ferner ist die Vorrichtung eine Vorrichtung zur präzisen und wiederholt lösbaren Verankerung von Objekten an einem Zahnbogen eines Probanden zur Erfassung von Position und oder Bewegung des Zahnbogens bzw. des Unterkiefers, insbesondere relativ zum Oberkiefer. Ferner weist die Vorrichtung gemäß der dritten Ausführungsform die folgenden weiteren Merkmale auf zwei Kontaktbereiche, die vorn (43) und hinten (44) vorhanden sind; einen Haltebogen (4) mit konkaven Kontaktflächen (41) im vorderem Kontaktbereich (43) und mit konkaven Kontaktflächen (41) im hinteren Kontaktbereich (44) und mit einem Abstand (45) zwischen hinterem und vorderem Kontaktbereich und seitliche mechanische Anpressung der Kontaktflächen (41) auf den Zahnbogen, wobei der Abstand HA (45) insbesondere größer als 20mm ist.

Eine vierte Ausführungsform der Vorrichtung umfasst die Merkmale einer der ersten bis dritten Ausführungsformen, wobei zwischen den vorderen und hinteren Kontaktbereichen (43) und (44) ein Abstandsbereich (45) vorhanden ist, in dem der Haltebogen (4) nicht oder nur mit geringer Klemmkraft am Zahnbogen (1) anliegt.

Eine fünfte Ausführungsform der Vorrichtung umfasst die Merkmale einer der ersten bis vierten Ausführungsformen, wobei am Haltebogen (4) mindestens eine mechanische Schnittstelle (5) vorhanden ist, mit Befestigungsvorrichtung für technische Baugruppen, insbesondere Sensoren (61) , Signalgeber, Marker (62) etc.

Eine sechste Ausführungsform der Vorrichtung umfasst die Merkmale einer der ersten bis fünften Ausführungsformen, wobei an Stümpfen im Zahnbogen, die unterhalb der Okklusionsebene liegen, die Haltevorrichtung den Stumpf überdeckt und umgreift.

Eine siebte Ausführungsform der Vorrichtung umfasst die Merkmale einer der ersten bis sechsten Ausführungsformen, wobei an endständigen Objekten im Zahnbogen, insbesondere an vereinzelten Molaren oder Implantaten, die Haltevorrichtung den Stumpf umgreift.

Ferner wird eine erste Ausführungsform eines Verfahrens zum Herstellen einer der Vorrichtungen der ersten bis siebten Ausführungsformen der Vorrichtung bereitgestellt, bei dem
- zunächst ein realer Zahnbogen (1) bestehend aus einer Reihe von Objekten ausgewählt aus (Zahn, Krone, Füllung, Implantat, Zahnstumpf, Zahnfleisch) abgeformt und in einen 3D Datensatz eines Zahnbogens (3) übertragen wird;
- dann der 3D Datensatz des Zahnbogens (3) gezielt virtuell bearbeitet wird, um einen umgearbeiteten 3D Datensatz des Zahnbogens (30) mit insbesondere zwei Klemmbereiche 43 und 44 zu erzeugen;
- dann eine Verschmelzung aus virtuellem Zahnbogen (30) und Haltebogen Preform (70) vorgenommen wird, woraus der virtuelle Haltebogen (40) hervorgeht; und
   dann eine 3D CAM formgebendes Verfahren benutzt wird, um den realen Haltebogen (4) herzustellen, insbesondere 3D Druck oder 3D Fräsen.

Eine zweite Ausführungsform des Verfahrens umfasst die Schritte der ersten Ausführungsform des Verfahrens, wobei zwischen den vorderen und hinteren Kontaktbereichen (43) und (44) ein Abstandsbereich (46) erzeugt wird durch Addition einer Dickschicht (33) mit Dicke D zwischen 0,1 und 2,0 mm auf der Oberfläche des realen oder des virtuellen Zahnmodells im Bereich (46) und anschließend erzeugen eines 3D Modells des Haltebogens unter Verwendung der Verschmelzung von virtuellem Zahnbogen (3) und Dickschicht (33).

Eine dritte Ausführungsform des Verfahrens umfasst die Schritte der ersten oder zweiten Ausführungsform des Verfahrens wobei das virtuelle 3D Modell des Zahnbogens partiell oder ganz volumetrisch aufgeblasen wird, so dass dadurch ein verändertes virtuelles 3D Modell des Zahnbogens (30) erzeugt wird und so dass dadurch nach Erzeugung eine virtuellen Haltebogens (49) ein realer Haltebogen (4) mit Abstandsspalt in den Bereichen (46) mittels 3D Formgebung, insbesondere durch Digitaldruck erzeugt wird.

Eine vierte Ausführungsform des Verfahrens umfasst die Schritte einer der ersten bis dritten Ausführungsformen des Verfahrens wobei zwischen den vorderen und hinteren Kontaktbereichen (43) und (44) ein Abstandsbereich (46) erzeugt wird durch Addition einer Dickschicht (33) mit Dicke D zwischen 0,1 und 2,0 mm auf der Oberfläche des realen oder des virtuellen Zahnmodells im Bereich (46) und das Verfahren anschließend den Schritt des Erzeugens eines 3D Modells des Haltebogens unter Verwendung der Verschmelzung von virtuellem Zahnbogen (3) und Dickschicht (33) umfasst.

### Liste der Ziffern und Nummerierung

### Realer Zahnbogen

11 reale Kontaktflächen
1.6 Okklusionsfläche Molar
12 Okklusionsflächen des Zahnbogens
1.61 Zahnwurzel
1.62 Zahnfleisch
Kiefer, Unterkiefer
Virtueller 3D Zahnbogen original
erste Hälfte 3D Zahnbogen
zweite Hälfte 3D Zahnbogen
30 Virtueller veränderter 3D Zahnbogen mit Spaltform bei 46
31 Kontaktfläche am 3D Zahnbogen, 31.1 ... 31.4
33 Abstandsmittel zur Erzeugung der Abstandsbereiche bei 46
4 Realer Haltebogen
40 virtueller 3D Haltebogen
41 Kontaktflächen des Haltebogens in den Bereichen 43 und 44, 41.1 ... 41.4
41.7 Abstandsfläche im Bereich 46
46.1 Aufgetragene Schichtdicke als Abstandsmittel im virtuellen 3D Modell
46.7 3D Erzeugung der Abstandsfläche bei 46
43 Vorderer Kontaktbereich
44 Hinterer Kontaktbereich
45 Abstand zwischen den Kontaktbereichen
46 Abstandsbereich
5 mechanische Schnittstelle
6 Sensorik und Instrumentierung
61 eingebauter Signalgeber
62 direkt montierter Marker oder LED
70 3d Modell virtueller Haltebogen Preform

## Patentansprüche

1. Vorrichtung (4) zur reversibel lösbaren Befestigung an Zähnen (1) eines Oberkiefers oder Unterkiefers, aufweisend:
konkave Oberflächenbereiche (41.1-41.4), die jeweils zum Kontaktieren von einem lateralen, insbesondere bukkalen, Oberflächenbereich eines Zahnes (1) ausgebildet sind, um jeweils einen Formschluss zu bilden,
wobei Kauflächen (1.6) sämtlicher Zähne (1) frei bleiben, wenn die Vorrichtung an den Zähnen befestigt ist, sodass eine Kaubewegung nicht behindert ist,
wobei die Oberflächenbereiche (41.1-41.4) der Vorrichtung und die Oberflächenbereiche der Zähne (1) jeweils durch eine elastomechanische Kraft in Kontakt gebracht werden können und mittels der elastomechanischen Kraft gehalten werden können, wenn die Vorrichtung an den Zähnen befestigt ist,
wobei die Vorrichtung (4) eine U-Form hat,
wobei die elastomechanische Kraft eine elastische Rückstellkraft durch Verformung der Vorrichtung (4) ist, und die Zähne (1) vermeiden, dass ein elastisch verformter Bereich der Vorrichtung wieder seine ursprüngliche nicht-deformierte Form annimmt, wenn die Vorrichtung an den Zähnen befestigt ist,
**dadurch gekennzeichnet, dass** die Vorrichtung (4) zum Anbringen an die Zähne an den Enden des U's leicht aufbiegbar bzw. spreizbar ist.

2. Vorrichtung gemäß einem der vorangehenden Ansprüche, wobei die Oberflächenbereiche (41.1-41.4) der Vorrichtung (4) und die Oberflächenbereiche der Zähne (1) zumindest abschnittsweise komplementäre Oberflächenform aufweisen.

3. Vorrichtung gemäß einem der vorangehenden Ansprüche, wobei die Oberflächenbereiche (41.1-41.4) der Vorrichtung zumindest drei, insbesondere vier, konkave Oberflächenbereiche (41.1-41.4) der Vorrichtung (4) aufweisen, insbesondere einen linken vorderen, einen linken hinteren, einen rechten vorderen und einen rechten hinteren Oberflächenbereich,
wobei zwischen den vorderen und hinteren Oberflächenbereichen (41.1-41.4) jeweils ein Zwischenabschnitt (46) der Vorrichtung (4) vorgesehen ist, in dem keine Kontaktbereiche zu Zähnen (1) vorhanden sind oder nur form- und/oder kraftschlussfreie Kontaktbereiche zu Zähnen (1) vorhanden sind.

4. Vorrichtung gemäß einem der vorangehenden Ansprüche, wobei ein Abstand zwischen den vorderen und hinteren Oberflächenbereichen (41.1-41.4) jeweils 0,1 bis 0,6, insbesondere 0,2 bis 0,4, mal eine Gebißausdehnung von vorn nach hinten beträgt.

5. Vorrichtung gemäß einem der vorangehenden Ansprüche, wobei zumindest einer, insbesondere alle, der Oberflächenbereiche (41.1-41.4) der Vorrichtung (4) konkav in zwei quer, insbesondere senkrecht, zueinander verlaufenden Richtungen ist.

6. Vorrichtung gemäß einem der vorangehenden Ansprüche, wobei die Vorrichtung (4) ferner eine Befestigungsmöglichkeit (5) für ein Element, insbesondere eine Markierung und/oder einen Signalgeber und/oder einen Sensor und/oder eine Lichtquelle und/oder einen Bügel, bereitstellt.

7. Vorrichtung gemäß einem der vorangehenden Ansprüche, wobei die Befestigungsmöglichkeit (5) als ein im Bereich der Schneidezähne angeordneter nach vorn hervorstehender Arm ausgebildet ist.

8. Vorrichtung gemäß einem der vorangehenden Ansprüche, wobei die Vorrichtung (4) aus einem elastischen Material, insbesondere Kunststoff gefertigt ist und/oder wobei die Vorrichtung (4) mehrteilig, insbesondere zweiteilig, ausgebildet ist und ferner mindestens ein Kopplungselement vorgesehen ist, mit Hilfe dessen die mehreren Teile der Vorrichtung gekoppelt werden können, während die Vorrichtung an den Zähnen befestigt ist, wobei durch Kopplung der mehreren Teile der Vorrichtung eine Anpresskraft auf die Oberflächenbereiche der Vorrichtung ausgeübt wird, die diese an die lateralen Oberflächenbereiche der Zähne presst, sodass der Formschluss erfolgt.

9. Verfahren zur Herstellung einer Vorrichtung (4) zur reversibel lösbaren Befestigung an Zähnen (1) eines Oberkiefers oder Unterkiefers, wobei das Verfahren aufweist:
Bilden von konkaven Oberflächenbereichen (41.1-41.4), die jeweils zum Kontaktieren von einem lateralen, insbesondere bukkalen, Oberflächenbereich eines Zahnes ausgebildet sind, um jeweils einen Formschluss zu bilden,
wobei Kauflächen (1.6) sämtlicher Zähne frei bleiben, wenn die Vorrichtung an den Zähnen befestigt ist, sodass eine Kaubewegung nicht behindert ist,
wobei die Oberflächenbereiche (41.1-41.4) der Vorrichtung und die Oberflächenbereiche der Zähne jeweils durch eine elastomechanische Kraft in Kontakt gebracht werden können und mittels der elastomechanischen Kraft gehalten werden können, wenn die Vorrichtung an den Zähnen befestigt ist,
wobei die Vorrichtung (4) eine U-Form hat,
wobei die elastomechanische Kraft eine elastische Rückstellkraft durch Verformung der Vorrichtung (4) ist, und die Zähne (1) vermeiden, dass ein elastisch verformter Bereich der Vorrichtung wieder seine ursprüngliche nicht-deformierte Form annimmt, wenn die Vorrichtung an den Zähnen befestigt ist,
wobei die Vorrichtung (4) zum Anbringen an die Zähne an den Enden des U's leicht aufbiegbar bzw. spreizbar ist.

10. Verfahren gemäß dem vorangehenden Anspruch, wobei das Verfahren ferner aufweist:
Bereitstellen eines 3D-Modells (3) der Zähne, das die lateralen zur Kontaktierung mit der Vorrichtung vorgesehenen Oberflächenbereiche (31.1-31.4) der Zähne des Oberkiefers oder des Unterkiefers umfasst;
Modifizieren des 3D-Modells durch Vergrößern in Bereichen außerhalb der zur Kontaktierung vorgesehenen lateralen Oberflächenbereiche;
Definieren der Form der Vorrichtung durch Komplementbildung des modifizierten Modells;

11. Verfahren gemäß einem vorangehenden Ansprüche 9 oder 10, wobei das Definieren der Form der Vorrichtung ferner ausgehend von einem U-förmigen Rohling erfolgt.

12. Verfahren gemäß einem vorangehenden Ansprüche 9 bis 11, wobei das Modifizieren des 3D-Modells ein Definieren eines Abstandes von nicht zur Kontaktierung vorgesehenen Oberflächenbereichen der Zähne zu nicht zur Kontaktierung vorgesehenen Oberflächenbereichen der Vorrichtung zwischen 0,1 mm und 2 mm umfasst.

13. Verfahren gemäß einem vorangehenden Ansprüche 9 bis 12,
wobei das 3D-Modell als reales körperliches Objekt oder als elektronischer Datensatz bereitgestellt wird,
wobei das Modifizieren am realen körperlichen Objekt oder mittels einer den elektronischen Datensatz darstellenden Visualisierungssoftware erfolgt,
wobei das Definieren der Form der Vorrichtung durch physikalischen Abdruck des modifizierten Modells oder Komplementbildung bzw. Differenzbildung zwischen dem Rohling und dem modifizierten Modell in einer elektronischen Datenverarbeitungsanlage erfolgt.

14. Verfahren gemäß einem vorangehenden Ansprüche 9 bis 13, ferner aufweisend:
Ausgeben eines die Form der Vorrichtung definierenden Datensatzes an ein System, das zur additiven Fertigung geeignet ist, zum Beispiel an einen 3D-Drucker; und
Herstellen der Vorrichtung mittels des Systems, zum Beispiel des 3D-Druckers.

## Claims

1. Device (4) for reversibly releasable attachment to teeth (1) of an upper jaw or lower jaw, comprising:
concave surface regions (41.1-41.4), each designed for contacting a lateral, in particular buccal, surface region of a tooth (1) in order to form in each case a form-fit engagement,
wherein masticatory surfaces (1.6) of all the teeth (1) remain free when the device is attached to the teeth, such that a chewing movement is not impeded,
wherein the surface regions (41.1-41.4) of the device and the surface regions of the teeth (1) can respectively be brought into contact by an elastomechanical force and retained by means of the elastomechanical force when the device is attached to the teeth,
wherein the device (4) has a U shape,
wherein the elastomechanical force is an elastic restoring force owing to deformation of the device (4), and the teeth (1) prevent an elastically deformed region of the device from readopting its original undeformed shape when the device is attached to the teeth,
**characterized in that** the device (4) can be slightly bent open or spread apart at the ends of the "U" for mounting on the teeth.

2. Device according to one of the preceding claims, wherein the surface regions (41.1-41.4) of the device (4) and the surface regions of the teeth (1) have a complementary surface shape at least in certain portions.

3. Device according to either of the preceding claims, wherein the surface regions (41.1-41.4) of the device have at least three, in particular four, concave surface regions (41.1-41.4) of the device (4), in particular a left anterior, a left posterior, a right anterior and a right posterior surface region,
wherein provided between the anterior and posterior surface regions (41.1-41.4) is a respective intermediate portion (46) of the device (4) in which there are no regions of contact with teeth (1) or in which there are only regions of contact with teeth (1) that do not have a form-fit and/or force-fit engagement.

4. Device according to one of the preceding claims, wherein a spacing between the anterior and posterior surface regions (41.1-41.4) is in each case 0.1 to 0.6, in particular 0.2 to 0.4, times a dentition range from front to rear.

5. Device according to one of the preceding claims, wherein at least one of the surface regions (41.1-41.4) of the device (4), in particular all of the surface regions of the device, is/are concave in two directions extending transversely, in particular perpendicularly, in relation to each other.

6. Device according to one of the preceding claims, wherein the device (4) moreover provides an attachment option (5) for an element, in particular a marker and/or a signal transmitter and/or a sensor and/or a light source and/or a bow.

7. Device according to one of the preceding claims, wherein the attachment option (5) is in the form of a forwardly protruding arm located in the region of the incisors.

8. Device according to one of the preceding claims, wherein the device (4) is manufactured from an elastic material, in particular plastic, and/or wherein the device (4) is formed in multiple parts, in particular in two parts, and moreover provided is at least one coupling element, by means of which the multiple parts of the device can be coupled while the device is attached to the teeth, wherein the coupling of the multiple parts of the device causes a contact pressure to be exerted on the surface regions of the device, the contact pressure pressing these onto the lateral surface regions of the teeth, resulting in the form-fit engagement.

9. Method for producing a device (4) for reversibly releasable attachment to teeth (1) of an upper jaw or lower jaw, wherein the method comprises:
forming concave surface regions (41.1-41.4), each of which is designed for contacting a lateral, in particular buccal, surface region of a tooth in order to form in each case a form-fit engagement,
wherein masticatory surfaces (1.6) of all the teeth remain free when the device is attached to the teeth, such that a chewing movement is not impeded,
wherein the surface regions (41.1-41.4) of the device and the surface regions of the teeth can respectively be brought into contact by an elastomechanical force and retained by means of the elastomechanical force when the device is attached to the teeth,
wherein the device (4) has a U shape,
wherein the elastomechanical force is an elastic restoring force owing to deformation of the device (4), and the teeth (1) prevent an elastically deformed region of the device from readopting its original undeformed shape when the device is attached to the teeth,
wherein the device (4) can be slightly bent open or spread apart at the ends of the "U" for mounting on the teeth.

10. Method according to the preceding claim, wherein the method moreover comprises:
making available a 3D model (3) of the teeth, which model comprises the lateral surface regions (31.1-31.4), of the teeth of the upper jaw or lower jaw, that are provided for making contact with the device;
modifying the 3D model by enlargement in regions outside the lateral surface regions provided for making contact;
defining the shape of the device by complement formation of the modified model.

11. Method according to either of the preceding claims 9 and 10, wherein the definition of the shape of the device moreover proceeds from a U-shaped blank.

12. Method according to one of the preceding claims 9 to 11, wherein the modification of the 3D model comprises defining a spacing of between 0.1 mm and 2 mm between surface regions of the teeth that are not provided for making contact and surface regions of the device that are not provided for making contact.

13. Method according to one of the preceding claims 9 to 12,
wherein the 3D model is made available as a real physical object or as an electronic data set,
wherein the modification is performed on the real physical object or by means of visualization software representing the electronic data set,
wherein the shape of the device is defined by taking a physical impression of the modified model or by complement formation or forming a difference between the blank and the modified model in an electronic data processor.

14. Method according to one of the preceding claims 9 to 13, moreover comprising:
outputting a data set defining the shape of the device to a system, which is suitable for additive manufacture, for example to a 3D printer; and
producing the device by means of the system, for example the 3D printer.

## Revendications

1. Dispositif (4) servant à la fixation amovible de manière réversible aux dents (1) d'une mâchoire supérieure ou d'une mâchoire inférieure, présentant :
des régions de surface concaves (41.1-41.4) qui sont formées respectivement pour entrer en contact avec une région de surface latérale, en particulier buccale, d'une dent (1), afin de former respectivement un engagement par complémentarité de formes,
les surfaces de mastication (1.6) de toutes les dents (1) demeurant libres lorsque le dispositif est fixé aux dents, de sorte qu'un mouvement de mastication ne soit pas gêné,
les régions de surface (41.1-41.4) du dispositif et les régions de surface des dents (1) pouvant être amenées en contact respectivement par une force élastomécanique et pouvant être retenues au moyen de la force élastomécanique lorsque le dispositif est fixé aux dents, le dispositif (4) ayant une forme en U,
la force élastomécanique étant une force de rappel élastique par déformation du dispositif (4), et les dents (1) empêchant qu'une région déformée élastiquement du dispositif n'adopte à nouveau sa forme non déformée initiale lorsque le dispositif est fixé aux dents,
**caractérisé en ce que**
le dispositif (4) peut être ouvert par flexion ou peut être écarté légèrement aux extrémités du U pour le placement sur les dents.

2. Dispositif selon l'une des revendications précédentes, les régions de surface (41.1-41.4) du dispositif (4) et les régions de surface des dents (1) présentant une forme de surface complémentaire au moins dans certaines parties.

3. Dispositif selon l'une des revendications précédentes, les régions de surface (41.1- 41.4) du dispositif présentant au moins trois, en particulier quatre régions de surface concaves (41.1-41.4) du dispositif (4), en particulier une région de surface avant gauche, une région de surface arrière gauche, une région de surface avant droite et une région de surface arrière droite,
une partie intermédiaire (46) du dispositif (4) étant prévue respectivement entre les régions de surface avant et arrière (41.1-41.4), partie intermédiaire dans laquelle aucunes régions de contact avec les dents (1) ne sont présentes ou seulement des régions de contact sans engagement par complémentarité de formes et/ou à force avec les dents (1) sont présentes.

4. Dispositif selon l'une des revendications précédentes, un espacement entre les régions de surface avant et arrière (41.1-41.4) valant respectivement de 0,1 à 0,6, en particulier de 0,2 à 0,4 fois une dimension de denture de l'avant vers l'arrière.

5. Dispositif selon l'une des revendications précédentes, au moins une, en particulier toutes les régions de surface (41.1-41.4) du dispositif (4) étant concaves dans deux directions s'étendant transversalement, en particulier perpendiculairement, l'une par rapport à l'autre.

6. Dispositif selon l'une des revendications précédentes, le dispositif (4) fournissant en outre une possibilité de fixation (5) pour un élément, en particulier un marquage et/ou un transmetteur de signaux et/ou un capteur et/ou une source de lumière et/ou un étrier.

7. Dispositif selon l'une des revendications précédentes, la possibilité de fixation (5) étant réalisée sous la forme d'un bras faisant saillie vers l'avant et disposé dans la région des incisives.

8. Dispositif selon l'une des revendications précédentes, le dispositif (4) étant fabriqué à partir d'une matière élastique, en particulier d'une matière synthétique et/ou le dispositif (4) étant réalisé en plusieurs parties, en particulier en deux parties, et en outre au moins un élément d'accouplement étant prévu, à l'aide duquel les plusieurs parties du dispositif peuvent être accouplées pendant que le dispositif est fixé aux dents et, par accouplement des plusieurs parties du dispositif, une force de pression étant exercée sur les régions de surface du dispositif, laquelle presse celles-ci contre les régions de surface latérales des dents, de sorte que l'engagement par complémentarité de formes soit effectué.

9. Procédé de fabrication d'un dispositif (4) servant à la fixation amovible de manière réversible aux dents (1) d'une mâchoire supérieure ou d'une mâchoire inférieure, le procédé présentant :
la formation de régions de surface concaves (41.1-41.4) qui sont formées respectivement pour entrer en contact avec une région de surface latérale, en particulier buccale, d'une dent, afin de former respectivement un engagement par complémentarité de formes,
les surfaces de mastication (1.6) de toutes les dents demeurant libres lorsque le dispositif est fixé aux dents, de sorte qu'un mouvement de mastication ne soit pas gêné,
les régions de surface (41.1-41.4) du dispositif et les régions de surface des dents pouvant être amenées en contact respectivement par une force élastomécanique et pouvant être retenues au moyen de la force élastomécanique lorsque le dispositif est fixé aux dents, le dispositif (4) ayant une forme en U,
la force élastomécanique étant une force de rappel élastique par déformation du dispositif (4), et les dents (1) empêchant qu'une région déformée élastiquement du dispositif n'adopte à nouveau sa forme non déformée initiale lorsque le dispositif est fixé aux dents,
le dispositif (4) pouvant être ouvert par flexion ou pouvant être écarté légèrement aux extrémités du U pour le placement sur les dents.

10. Procédé selon la revendication précédente, le procédé présentant en outre :
la fourniture d'un modèle 3D (3) des dents, qui comprend les régions de surface (31.1-31.4) latérales, prévues pour l'entrée en contact avec le dispositif, des dents de la mâchoire supérieure ou de la mâchoire inférieure ;
la modification du modèle 3D par agrandissement dans des régions à l'extérieur des régions de surface latérales prévue pour l'entrée en contact ;
la définition de la forme du dispositif par formation complémentaire du modèle modifié.

11. Procédé selon l'une des revendications précédentes 9 ou 10, la définition de la forme du dispositif étant effectuée en outre à partir d'une ébauche en forme de U.

12. Procédé selon l'une des revendications précédentes 9 à 11, la modification du modèle 3D comprenant une définition d'un espacement d'entre 0,1 mm et 2 mm entre des régions de surface des dents non prévues pour l'entrée en contact et des régions de surface du dispositif non prévues pour l'entrée en contact.

13. Procédé selon l'une des revendications précédentes 9 à 12,
le modèle 3D étant fourni en tant qu'objet physique réel ou en tant qu'ensemble de données électronique,
la modification étant effectuée sur l'objet physique réel ou au moyen d'un logiciel de visualisation représentant l'ensemble de données électronique,
la définition de la forme du dispositif étant effectuée en prenant une empreinte physique du modèle modifié ou par formation complémentaire ou formation différentielle entre l'ébauche et le modèle modifié dans un système de traitement de données électronique.

14. Procédé selon l'une des revendications précédentes 9 à 13, présentant en outre :
la délivrance d'un ensemble de données définissant la forme du dispositif à un système qui est approprié à la fabrication additive, par exemple à une imprimante 3D ; et
la fabrication du dispositif au moyen du système, par exemple de l'imprimante 3D.
